# EUROPEAN PATENT APPLICATION

(11) **EP 2 127 693 A1**
(43) Date of publication of application: **02.12.2009**
(21) Application number: 08704099.4
(22) Date of filing: 29.01.2008
(51) Int. Cl.: A61M 37/00, B29C 67/00

(54) **PROCESS FOR PRODUCING MICRONEEDLE OF THERMOSENSITIVE SUBSTANCE**

(30) Priority: 29.01.2007 JP 2007018006
(71) Applicant: Medrx Co., Ltd., Higashikagawa-shi Kagawa 769-2712 (JP)
(72) Inventor: HAMAMOTO, Hidetoshi, Higashikagawa-shi Kagawa 769-2712 (JP); ISHIBASHI, Masaki, Higashikagawa-shi Kagawa 769-2712 (JP); KOBAYASHI, Katsunori, Higashikagawa-shi Kagawa 769-2712 (JP)
(74) Representative: Jackson, Martin Peter
(86) International application number: PCT/JP2008/051317
(87) International publication number: WO 2008/093679

(57) **Abstract**

Provided is a novel method of producing a microneedle at low cost and on a mass-production scale.

A jig is heated to a temperature above the level at which a temperature-sensitive material that exhibits thermoplastic deformation becomes viscoplastic, and the jig is brought into contact with the temperature-sensitive material, thereafter the jig is pulled part from the temperature-sensitive material to elongate the portion of the temperature-sensitive material in contact with the jig, whereby acicular projections are formed. The method of microneedle production of the present invention preferably comprises the steps (i) to (iv) below:
(i) the step of setting a jig temperature above the temperature at which a temperature-sensitive material that exhibits thermoplastic deformation becomes viscoplastic,
(ii) the step of bringing the jig into contact with the temperature-sensitive material to make the temperature-sensitive material at the contact portion viscoplastic,
(iii) the step of pulling the jig from the temperature-sensitive material, and elongating the temperature-sensitive material in a viscoplastic state into thread forms,
(iv) the step of discontinuing the pulling of the jig from the temperature-sensitive material or decelerating the pulling to cut the thread-like temperature-sensitive material and form acicular projections.

## Description

### TECHNICAL FIELD OF THE INVENTION

The present invention relates to a microneedle and a method of producing the same, more specifically to a method of microneedle production applicable to any solid substance that becomes viscoplastic at increased temperature. The present invention also relates to a method that enables easy production of multiple microneedles of temperature-sensitive material formed in an array (i.e., microneedles comprising a plurality of acicular projections arranged in an array).

### BACKGROUND OF THE INVENTION

Transdermal administration of a drug has been achieved normally using a transdermal preparation in the form of solutions or ointments applied or plastered to the skin surface. The human skin normally comprises the stratum corneum, which has a laminar structure 10 to 30 µm in thickness, the epidermal tissue layer, which is about 70 µm in thickness (hereinafter also simply referred to as "epidermal layer"), and the dermal tissue layer, which is about 2 mm in thickness (hereinafter also simply referred to as "dermal layer").

The stratum corneum, a laminar structure constituting the outermost layer of the skin, functions as a barrier to prevent various drugs from penetrating the skin. Generally, about 50 to 90% of the barrier action of the skin is attributed to the stratum corneum. Located under the stratum corneum, the epidermal layer accounts for the remaining 10% or more of the skin barrier action. Meanwhile, in the dermal layer under the epidermal layer, an extensive capillary network is present in the vicinity of the junction between the dermal layer and the epidermal layer. Once a drug reaches a depth of the dermis (i.e., upon entry in the dermal layer), the drug quickly diffuses in deeper tissues (hair follicles, muscles and the like) via the network. Then, the drug is systematically diffused through the blood circulation from the capillaries.

Therefore, to increase the skin permeability of a drug, it is necessary to maximize the efficiency of the delivery of the drug to the dermal layer. Accordingly, as shown in JP-A-2006-149818, it has been a general practice to date that the stratum corneum is topically destroyed using a microneedle or a microsyringe to forcibly deliver the drug into the dermal layer.

JP-A-2006-513811 discloses several examples of typical dimensions of microneedles, including a length of 1 to 3 µm by a root diameter of 10 nm to 1 mm.

A wide variety of methods are available for microneedle production, including those disclosed in JP-A-2006-513811, wherein microneedles are molded, and those described in JP-A-2003-501163, wherein hollow microneedles are produced. However, these publicly known methods are said to have the drawback of difficulty in obtaining appropriate hardness; some needles produced are too soft, and others are too hard. Another drawback is high manufacturing cost. In the methods involving the use of a mold, the tips of microneedles chip easily because of the frictional stress exerted upon releasing the microneedle from the mold. For this reason, the reliability is poor in obtaining a uniform microarray (i.e., a plurality of microneedles arranged in an array).

Therefore, a problem to be solved in the currently available microneedles for transdermal absorption resides in how to prepare microneedles of biocompatible material possessing sufficient strength and durability. Furthermore, such microneedles are required to be produced at low cost because they are intended for single-use purposes.

### Disclosure of the Invention

### Problems to Be Solved by the Invention

In view of the circumstances described above, it is an object of the present invention to produce microneedles at low cost on a mass-production scale using a temperature-sensitive material, specifically to provide a novel method that enables production of microneedles at low cost on a mass-production scale.
It is another object of the present invention to provide a novel method of microneedle production that enables easy production of microneedles of high specification reliability with uniform tips (i.e., individual microneedles have chipping-free uniform tips).
It is still another object of the present invention to provide microneedles having chipping-free uniform tips, and permitting smooth penetration into the skin, and a method of producing the same.

### Means of Solving the Problems

The present inventors initially attempted to prepare uniform microneedles by casting a resin into a mold and releasing the mold, but were unable to obtain uniform microneedles at high percent yields because of insufficient casting into the mold, chipping during mold release and the like. The present inventors found, however, that microneedles with uniform tips can be produced, avoiding chipping, at low cost on a mass-production scale, by employing a mold-free process that can be described as "spontaneous shaping", which is apparently unlikely to produce uniform microneedles, compared to mold shaping.
Specifically, microneedles are produced from a temperature-sensitive material that changes from solid to viscoplastic with the rise of temperature, wherein a jig is heated to a temperature above the level for viscoplastic transformation of the temperature-sensitive material, and brought into contact with the temperature-sensitive material, and the jig is pulled away from the temperature-sensitive material, whereby the temperature-sensitive material bonded to the jig is elongated to form acicular projections. Hence, the present inventors found that the temperature-sensitive material, softened and elongated by the jig to become a thread-like state, forms acicular projections when the elongation is discontinued or decelerated, since the threads are cut by the effects of surface tension and the like.

Example procedures for the method of production are as follows:
(1) A temperature-sensitive material, extended in a film form, is attached to a vertically movable support with the film's temperature-sensitive material face down.
(2) Microneedles of silicon or metal for a jig are placed just under the support, and heated with a heat plate, electric heat wire or the like.
(3) The support is lowered to a position where the tips of the heated jig stops in the temperature-sensitive material film.
(4) The jig is heated at the position for a given time to soften the portions of the temperature-sensitive material in contact with the tips of the jig.
(5) As the support is pulled up at a constant rate, the softened portions of the temperature-sensitive material are pulled by the tips of the jig to form acicular projections on the temperature-sensitive material film. In this operation, desired acicular projections can be obtained by adjusting the pull-up rate in a single or two steps.
   The acicular projections thus formed are uniform in size, with nearly constant needle length, and do not undergo chipping at the tips thereof.
(6) The thus-obtained microarray of the temperature-sensitive material is cooled, and the temperature-sensitive material film is removed from the retaining substrate, whereby desired microneedles of the temperature-sensitive material are obtained.
   As required, the tips of the microneedles of the temperature-sensitive material can be made uniform by cutting the tips, or by immersing the tips in a solvent that dissolves the temperature-sensitive material.

The present inventors also found that hollow microneedles of temperature-sensitive material can be prepared through the steps (4) to (6) above, if the tips of the heated jig penetrate the temperature-sensitive material film and stop outside thereof to allow the tips of the jig to come in contact with the atmosphere in the step (3).
In the above embodiment, the temperature-sensitive material film is moved as fixed on the vertically movable support. The present inventors found that microneedles of temperature-sensitive material can also be obtained by vertically moving a jig while the temperature-sensitive material film is fixed.
As used herein, the term "vertical movement" of a jig or a temperature-sensitive material film refers to a movement in the perpendicular (gravitational) direction.
The present inventors also found that microneedles of temperature-sensitive material can also be obtained by arranging a temperature-sensitive material film and a jig on a straight line parallel to the horizontal plane, and moving at least one of the film and the jig on the straight line.

The findings described above disagree with the general notion that "articles of very small sizes can be shaped uniformly at high percent yields by using a mold as in the case of ordinary shaping." Rather, the present inventors have shown that mold-free shaping that can be described as spontaneous shaping produces uniform microneedles at high percent yields.
The inventors conducted further investigations based on these findings, and developed the present invention characterized as follows:

Accordingly, the present invention relates to:
[1] a method of producing a microneedle from a temperature-sensitive material that exhibits thermoplastic deformation, comprising:
   heating a jig to a temperature above the level for viscoplastic transformation of the temperature-sensitive material, bringing the jig into contact with the temperature-sensitive material, thereafter pulling the jig from the temperature-sensitive material, to elongate the temperature-sensitive material bonded to the jig, and to form acicular projections,
[2] a method of producing a microneedle, comprising the steps (i) to (iv) below:
   (i) the step of setting a jig at a temperature above the level for a viscoplastic state of a temperature-sensitive material that exhibits thermoplastic deformation,
   (ii) the step of bringing the jig into contact with the temperature-sensitive material to make the contact portion of the temperature-sensitive material viscoplastic,
   (iii) the step of pulling the jig apart from the temperature-sensitive material to elongate the temperature-sensitive material in the viscoplastic state in the form of threads, and
   (iv) the step for discontinuing the pulling or decelerating the pulling rate of the jig apart from the temperature-sensitive material to cut the threads of the temperature-sensitive material and form acicular projections,
[3] the method of claim 1 or 2, wherein either one of the temperature-sensitive material and the jig is placed on an upper portion of a straight line perpendicular to the horizontal plane, and the other on a lower portion, to allow the formation of acicular projections in the gravitational direction,
[4] the method of claim 1 or 2, wherein the temperature-sensitive material and the jig are arranged side by side on a straight line parallel to the horizontal plane, to allow the formation of acicular projections in the horizontal direction,
[5] the method of any one of [1] to [4] above, wherein the jig is a microneedle of silicon or metal,
[6] the method described in any one of [1] to [5] above, wherein the jig temperature is not lower than the glass transition point or melting point of the temperature-sensitive material,
[7] the method of any one of [1] to [6] above, wherein heating of the temperature-sensitive material by the jig lasts for 1 second to 1 minute,
[8] the method of any one of [1] to [7] above, wherein the pulling rate of the jig from the temperature-sensitive material for elongating the temperature-sensitive material into threads is 1 mm/minute to 100 mm/minute,
[9] the method of any one of [1] to [7] above, wherein the pulling rate of the jig from the temperature-sensitive material for elongating the temperature-sensitive material into threads is 2 mm/minute to 20 mm/minute,
[10] the method of any one of [2] to [9] above, wherein the pulling rate of the jig from the temperature-sensitive material for cutting the threads of the temperature-sensitive material is in the range from 0 mm/minute to half the pulling rate of the jig from the temperature-sensitive material for elongating the temperature-sensitive material into thread forms,
[11] the method of any one of [2] to [9] above, wherein the pulling the jig apart from the temperature-sensitive material is discontinued to cut the threads of the temperature-sensitive material,
[12] the method of any one of [1] to [11] above, wherein the temperature-sensitive material is a biodegradable resin or a polysaccharide,
[13] the method of [12] above, wherein the biodegradable resin is a polyfatty acid ester,
[14] the method of [13] above, wherein the polyfatty acid ester is polylactic acid, polyglycolic acid, or a lactic acid-glycolic acid copolymer,
[15] the method of [12] above, wherein the polysaccharide is maltose,
[16] the method of any one of [1] to [15] above, wherein the temperature-sensitive material has been shaped in a tabular form in advance,
[17] the method of any one of [1] to [16] above, further comprising immersing and dissolving the tips of the acicular projections in a solvent that dissolves the acicular projections,
[18] a microneedle of temperature-sensitive material obtained by the method of [1] above, each having an acicular projection whose root has a cross section corresponding to the cross section of the jig, whose tip has a pointed shape, and which has an arc extending from the tip to the root,
[19] the microneedle of [18] above, wherein the cross section of the root of the acicular projection is circular, polygonal, U-shaped or S-shaped,
[20] the microneedle of claim 17 or 18, wherein the temperature-sensitive material is a biodegradable resin or a polysaccharide,
[21] the microneedle of [20] above, wherein the biodegradable resin is a polyfatty acid ester,
[22] the microneedle of [21] above, wherein the polyfatty acid ester is polylactic acid, polyglycolic acid, or a lactic acid-glycolic acid copolymer,
[23] the microneedle of [20] above, wherein the polysaccharide is maltose,
[24] the microneedle of any one of [18] to [23] above, wherein acicular projections are provided on a tabular substrate,
[25] the microneedle of [24] above, having a plurality of acicular projections arranged in an array, and
[26] the microneedle of any one of [18] to [25] above, wherein the acicular projections are hollow acicular projections.

The present invention also encompasses the aspects shown below.
[27] A method of producing a hollow or non-hollow microneedle, wherein;
   (1) a microneedle of silicon or metal for a jig is set in place, and heated with a heat plate, electric heat wire or the like,
   (2) a sheet of temperature-sensitive material elongated into a film form is placed on a support that is movable in the vertical direction with respect to the plane on which the jig is present, and adjusted to a position just above the jig,
   (3) the temperature-sensitive material is moved to a position where the tips of the heated jig stops inside the temperature-sensitive material film, or to a position where the tips of the heated jig penetrate the temperature-sensitive material and stop outside thereof,
   (4) the jig is heated at that position for a given time to soften the portion of the temperature-sensitive material in contact with the tips of the jig,
   (5) thereafter, the jig is pulled apart from the temperature-sensitive material at a constant rate to cause the softened temperature-sensitive material to be pulled by the tips of the jig, and to form acicular projections on the temperature-sensitive material film, and
   (6) the microneedles of the temperature-sensitive material obtained in (5) are cooled, and removed from the substrate on which the temperature-sensitive material is present, to obtain a desired microneedle.
[28] A method of producing a hollow or non-hollow microneedle, wherein;
   (1) a sheet of temperature-sensitive material extended into a film form is set in place,
   (2) a microneedle of silicon or metal for a jig is placed on a support that is movable in the vertical direction with respect to the temperature-sensitive material film, and heated with a heat plate, electric heat wire or the like,
   (3) the jig is moved to a position where the tips of the heated jig stop inside the temperature-sensitive material film, or to a position where the tips of the heated jig penetrate the temperature-sensitive material and stop outside thereof,
   (4) the jig is heated at that position for a given time to soften the portion of the temperature-sensitive material in contact with the tips of the jig, (5) thereafter the jig is pulled apart from the temperature-sensitive material at a constant rate to cause the softened temperature-sensitive material to be pulled by the tips of the jig, and to form acicular projections on the temperature-sensitive material film, and (6) the microneedle of the temperature-sensitive material obtained in (5) are cooled, and removed from the substrate on which the temperature-sensitive material is present, to obtain a desired microneedle.

### Effect of the Invention

According to the method of production of the present invention, a temperature-sensitive material is softened and elongated in a viscoplastic state to thereby prepare microneedles of the temperature-sensitive material. As such, the method is simple and easily performable, thus reducing manufacturing cost considerably. The method also enables production of microneedles on a mass-production scale. The procedural simplicity ensures easy maintenance of product specifications and easy production of microneedles of high specification reliability.
The method also enables easy production of microneedles wherein the individual microneedles have chipping-free uniform tips, and which permit smooth penetration into the skin.
When a resin is used as the temperature-sensitive material in the method of production of the present invention, microneedles can be obtained with multiple drugs kneaded therein because the softening temperature of the resin is not so high.

### BRIEF DESCRIPTION OF THE DRAWINGS

FIG. 1 is a schematic diagram of a microneedle jig of silicon.
FIG. 2 is a schematic diagram at the start of softening or dissolution of a temperature-sensitive material layer.
FIG. 3 is a schematic diagram during softening or dissolution of a temperature-sensitive material layer.
FIG. 4 is a schematic diagram during formation of microneedles (acicular projections).
FIG. 5 is a schematic diagram at the end of formation of microneedles (acicular projections).
FIG. 6 is a schematic diagram at the start of softening or dissolution of a temperature-sensitive material layer in forming hollow microneedles (acicular projections).
FIG. 7 is a schematic diagram during formation of hollow microneedles (acicular projections).
FIG. 8 is a schematic diagram at the end of formation of hollow microneedles (acicular projections).
FIG. 9 is a light photomicrograph showing a magnified view of microneedle of polylactic acid.
FIG. 10 is a light photomicrograph showing a magnified view of microneedles of maltose.
FIG. 11 is a schematic diagram of a method wherein a jig is placed on an upper portion, and a temperature-sensitive material on a lower portion, to form acicular projections in the gravitational direction.
FIG. 12 is a schematic diagram of a method wherein a temperature-sensitive material and a jig are arranged on a straight line parallel to the horizontal plane, to form acicular projections in the direction parallel to the horizontal plane.
FIG. 13 is a light photomicrograph showing a magnified view of example microneedles prepared by the method of FIG. 11 (overall view).
FIG. 14 is a light photomicrograph showing a magnified view of example microneedles prepared by the method of FIG. 11 (magnified partial view).
FIG. 15 is a light photomicrograph showing a magnified view of example microneedles prepared by the method of FIG. 12 (overall view).
FIG. 16 is a light photomicrograph showing a magnified view of example microneedles prepared by the method of FIG. 12 (magnified partial view).
FIG. 17 is a light photomicrograph showing a magnified view of an acicular projection whose root has a square cross-section.
FIG. 18 is a light photomicrograph showing a magnified view of acicular projections whose root has a U-shaped cross-section (a plurality of projections).
FIG. 19 is a light photomicrograph showing a magnified view of an acicular projection whose root has a U-shaped cross-section (a single projection).

### [Caption for symbols]

1: acicular projection
2: substrate
3: temperature-sensitive material
4: heat plate
5: temperature-sensitive material in viscoplastic state
6, 6': acicular projection
7: support for temperature-sensitive material
8: opening
9: hollow portion
10A: basis
10, 10': microneedle
20: microarray

### Best Mode for Carrying out the Invention

The present invention is hereinafter described in detail with reference to preferred embodiments.
In the present invention, the term "temperature-sensitive material that exhibits thermoplastic deformation" refers to "a temperature-sensitive material that changes from solid to viscoplastic with the rise of temperature", exemplified by resins, saccharides (in particular, polysaccharides), mixtures thereof and the like. A preferable temperature-sensitive material is a highly biocompatible material; more preferably, a biodegradable material is chosen. The material is used normally after being shaped into a tabular form such as a chip, film or sheet.

Although the jig used in the present invention may be made of any material capable of maintaining a solid state at temperatures at which the temperature-sensitive material exhibits thermoplastic deformation, a silicon or metal jig is preferable. The shape of the jig is determined according to the number, shape etc. of the acicular projections of the microneedle to be produced.

In the present invention, a jig is brought into contact with a temperature-sensitive material, and the temperature-sensitive material at the contact portion is made viscoplastic, after which the jig is pulled apart from the contact portion to elongate the temperature-sensitive material in a viscoplastic state into threads, whereby acicular projections are formed. For this purpose, a jig prepared by a commonly used means is sufficient; examples include the wire cutting method described in JP-A-2006-513811, the mold method described in JP-A-2003-501163, the etching method described in US-A-2002/0082543 and the like.

In the present invention, the positional relationship between the temperature-sensitive material and the jig is not subject to limitations. Typical examples include an aspect wherein either a temperature-sensitive material or a jig is placed on an upper portion of a straight line perpendicular to the horizontal plane, i.e., in the perpendicular direction (gravitational direction), and the other on a lower portion, and an aspect wherein a temperature-sensitive material and a jig are arranged side by side on a straight line parallel to the horizontal plane.

The method of production of the present invention roughly comprises the three major processes described below.

### (First process)

The jig is heated to a temperature above the level at which the temperature-sensitive material becomes viscoplastic.

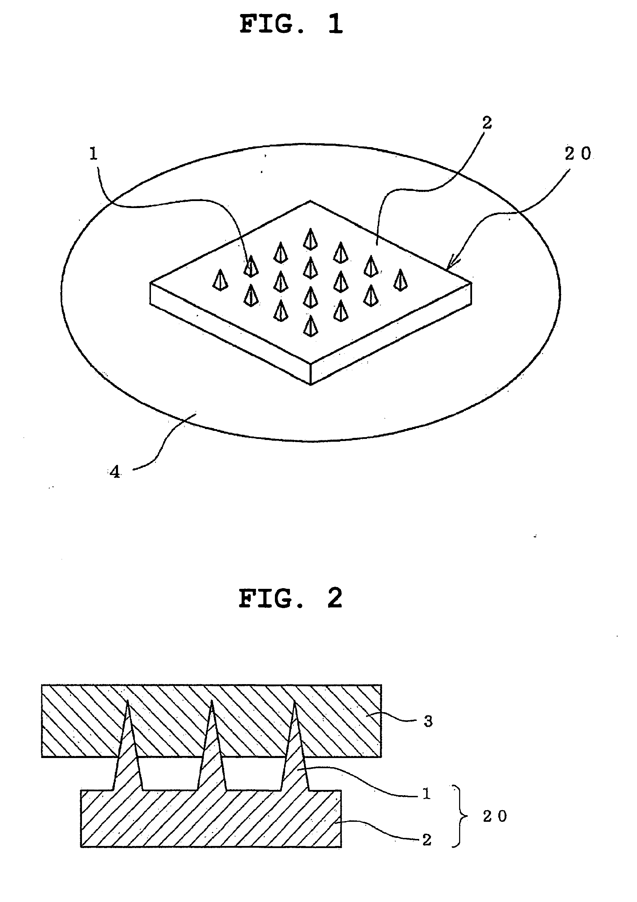

FIG. 1 shows an embodiment of the jig, wherein a plurality of acicular projections 1 are arranged in an array on a substrate 2 to form a silicon or metal microarray 20, which is heated using a heating means, for example, a heat plate, an electric heat wire or the like.

The jig is heated using a heating means such as a heat plate, in a way such that a portion of the jig to be contacted with the temperature-sensitive material (for example, acicular projections 1 in the microarray 20 of FIG. 1) has a temperature at which the temperature-sensitive material can be softened. Any publicly known heating means can be used.
In some cases, the temperature of the tips of the jig coming in contact with the temperature-sensitive material is preferably considerably higher than the glass transition point thereof to achieve uniform shaping of microneedles. However, because heating to the molten state of the temperature-sensitive material makes it difficult to pull out the microneedles, it is desirable to adjust the jig temperature and the duration of contact thereof with the temperature-sensitive material as appropriate.

The temperature-sensitive material used in the present invention is inserted into the skin and dwelled therein, and is desirably biocompatible, preferably both biocompatible and biodegradable. For this reason, biodegradable resins and polysaccharides are preferable. Examples of preferable biodegradable resins are aliphatic polyesters (polyfatty acid esters) such as polylactic acid, polyglycolic acid, and lactic acid-glycolic acid copolymers, with preference given to polylactic acid and lactic acid-glycolic acid copolymers. Polylactic acid is particularly preferable. Examples of polysaccharides include maltose, lactose, sucrose, mannitol, sorbitol and the like, maltose being particularly suitable.

A polylactic acid as mentioned herein has L-lactic acid and/or D-lactic acid as the main monomeric component. A polylactic acid with desired properties can be obtained by, for example, blending with lactic acid esters such as methyl lactate, butyl lactate, and hexadecyl lactate, or blending with additives that can be used in transdermal preparations, such as plasticizers and antioxidants.

A temperature of the heating means may be set as appropriate in consideration of the kind of the temperature-sensitive material, the material and size of jig, and the like. For example, when a heat plate is used to heat a silicon jig, and the heated jig is used to heat a polylactic acid having a weight-average molecular weight of 10,000 (temperature-sensitive material), the temperature of the heat plate preferably exceeds 60°C and is lower than 100°C, around 80°C is particularly suitable, because the glass transition point of a polylactic acid having a weight-average molecular weight of 10,000 is about 55°C. When the temperature of the heat plate is not more than 60°C, for example, even when the tips of the microneedle jig has reached the glass transition point of polylactic acid, it is cooled upon contact with the temperature-sensitive material (polylactic acid) and immediately becomes lower than the glass transition point of polylactic acid, making it difficult to pull out microneedles of the temperature-sensitive material, because the heat capacity of the jig is small. If the temperature of the heat plate is not less than 100°C, the tips of the microneedle jig become too hot, making it difficult to pull out temperature-sensitive material microneedles of desired length. When the temperature-sensitive material is maltose, whose melting point is 108°C, the temperature of the heat plate is preferably 130 to 170°C, more preferably about 150°C, in view of the heat capacity of the jig.

In the present invention, when the jig heating temperature is set at a relatively low temperature, the duration of contact of the jig and the temperature-sensitive material may be lengthened; when the jig heating temperature is set at a relatively high temperature, the duration of contact of the jig and the temperature-sensitive material may be shortened. Hence, by adjusting the duration of contact of the jig and the temperature-sensitive material as appropriate, a temperature-sensitive material in a suitable viscoplastic state can be formed.
Furthermore, by previously increasing the temperature of the temperature-sensitive material to reduce the temperature difference from the jig temperature, the temperature-sensitive material can be made viscoplastic in a short time. Therefore, by further shortening the duration of contact of the jig and the temperature-sensitive material, a temperature-sensitive material in a suitable viscoplastic state can be prepared.

### (Second process)

The heated jig is brought into contact with the temperature-sensitive material to make the temperature-sensitive material at the contact portion viscoplastic. For example, as shown in FIG. 2, the temperature-sensitive material is set to allow the tips of the acicular projections 1 of the heated microarray 20 to stop inside the temperature-sensitive material shaped in a tabular form (e.g., resin layer) 3. This is followed by heating for a given time to soften and melt the temperature-sensitive material around the jig to make the temperature-sensitive material viscoplastic.

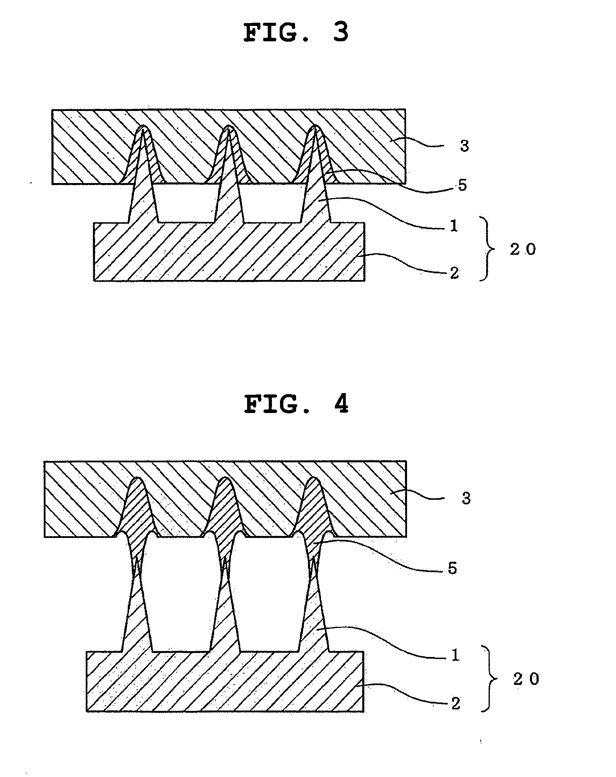

As shown in FIG. 3, as the duration of heating of the temperature-sensitive material with the jig (acicular projections 1 of microarray 20) increases, the amount of the temperature-sensitive material 5 in a viscoplastic state increases, and the amount of the temperature-sensitive material that forms desired acicular projections of microneedles of the temperature-sensitive material increases. Therefore, the lengths of the desired acicular projections in the microneedles of the temperature-sensitive material increases.

Although the contact time differs depending on the material (heat capacity) and shape of the jig, heating temperature, and the choice of temperature-sensitive material, it is preferable to keep the jig and the temperature-sensitive material in contact with each other for 1 second to 1 minute (preferably 5 seconds to 1 minute, more preferably 5 to 30 seconds). Hence, when the jig is a microarray 20, the acicular projections 1 of the microarray 20 are preferably retained in the temperature-sensitive material (e.g., resin layer) 3 for about 1 second to 1 minute (preferably 5 seconds to 1 minute, more preferably 5 to 30 seconds).

### (Third process)

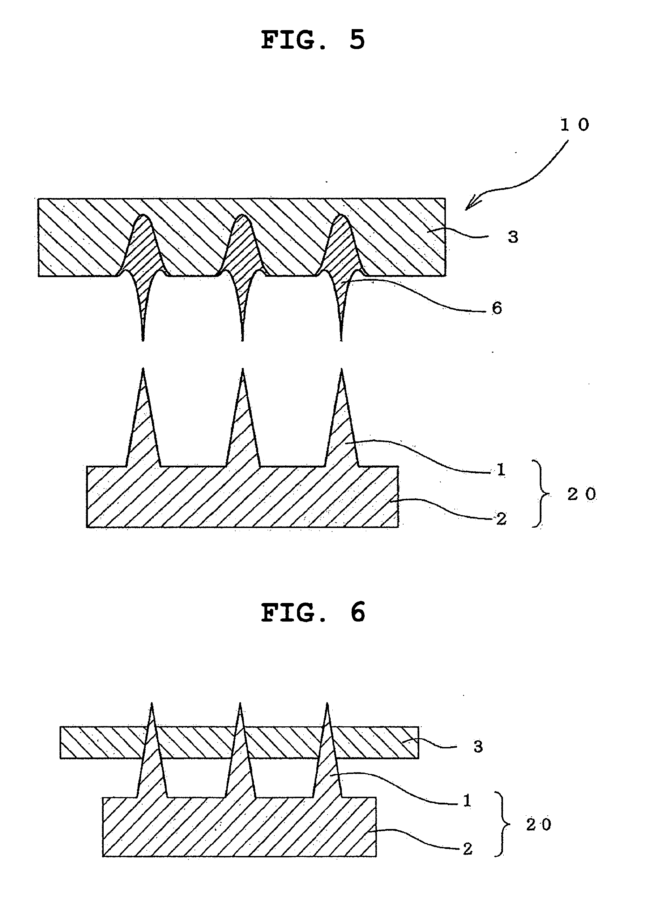

The jig is pulled apart from the temperature-sensitive material to elongate the temperature-sensitive material into thread forms.
Specifically, for example, as shown in FIG. 4, by pulling up at a constant rate the temperature-sensitive material (e.g., resin layer) 3 (or by lowering the microarray 20), the temperature-sensitive material 5 in a viscoplastic state is pulled by the tips of the acicular projections 1 of the microarray 20, and elongated into thread forms.
Then, the distance between the temperature-sensitive material (e.g., resin layer) 3 and the microarray 20 is further widened. As shown in FIG. 5, the temperature-sensitive material 5 in a viscoplastic state elongated into thread forms is cut, and a microneedle 10 having acicular projections 6 is completed.

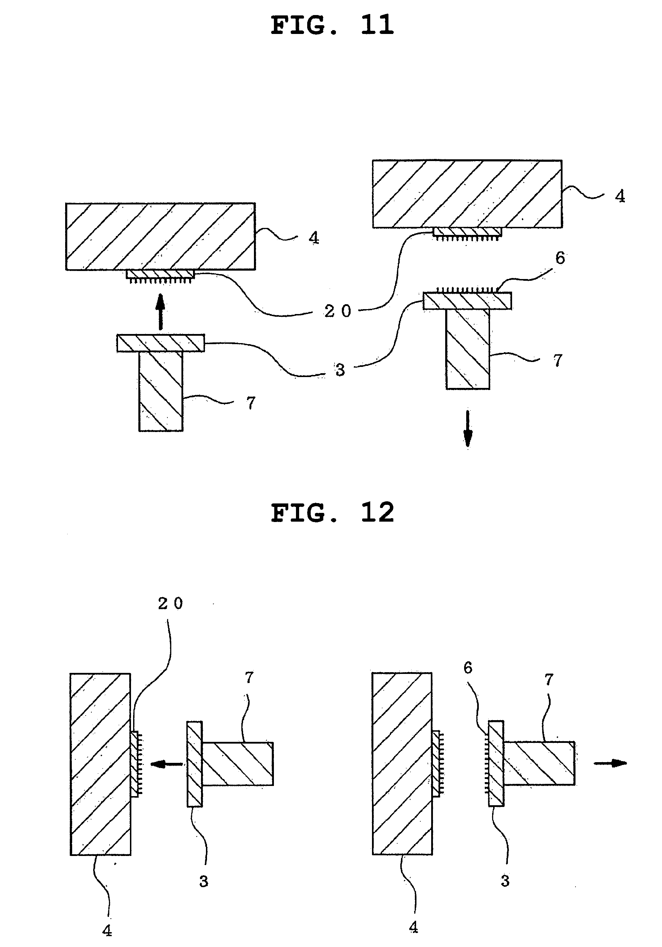

FIGS. 2 to 5 show embodiments wherein a temperature-sensitive material 3 is placed on an upper portion of a straight line in the vertical direction (in the perpendicular direction) to the horizontal plane, and a microarray 20 on a lower portion, and acicular projections 6 are formed in the gravitational direction. As shown in FIG. 11, however, it is also possible to place a jig (microarray 20) on an upper portion of a straight line perpendicular to the horizontal plane, and a temperature-sensitive material 3 on a lower portion, and to pull the jig (microarray 20) apart from the temperature-sensitive material 3 to form acicular projections 6 in the gravitational direction.

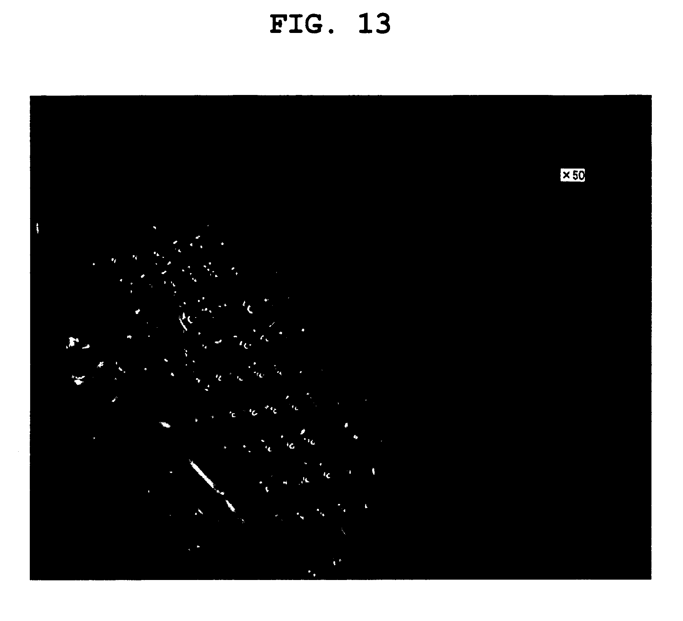

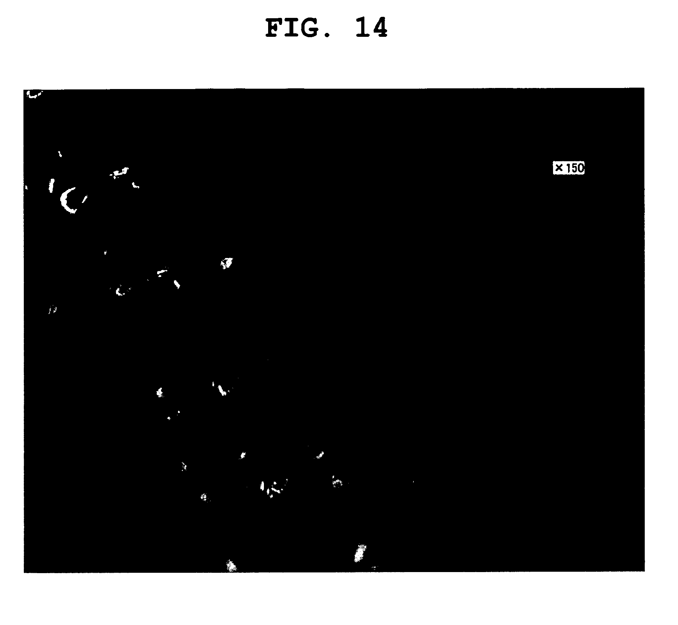

FIGS. 13 and 14 are light photomicrographs showing magnified views of a microneedle of biodegradable resin prepared by the above-described method (FIG. 13: overall view, FIG. 14: enlarged partial view).

It is also possible, as shown in FIG. 12, to form acicular projections 6 of temperature-sensitive material by placing a temperature-sensitive material 3 and a jig (microarray 20) on a straight line parallel to the horizontal plane, and moving either one of the temperature-sensitive material 3 and the jig (microarray 20) on the straight line to detach them.

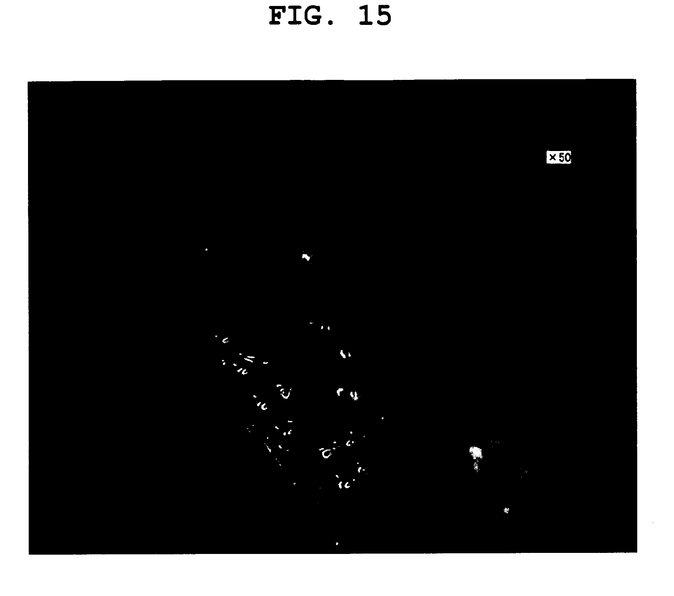

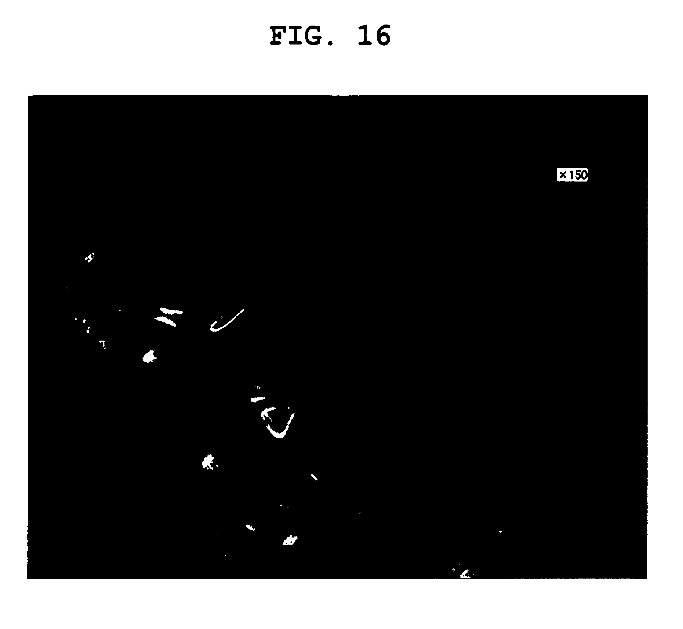

FIGS. 15 and 16 are light photomicrographs showing magnified views of a microneedle of biodegradable resin prepared by the above-described method (FIG. 15: overall view, FIG. 16: enlarged partial view).

In FIGS. 11 and 12, like FIGS. 2 to 5, the direction from the upper end of each figure to the lower end is taken as the gravitational direction, wherein the symbol 4 indicates a heat plate, the symbol 7 indicates a supporting member that supports a temperature-sensitive material 3, and the arrow indicates the direction of the movement of the temperature-sensitive material 3.

Both in the vertical (perpendicular) direction to the horizontal plane and in the parallel direction to the horizontal plane, the jig may be pulled apart from the temperature-sensitive material by moving both thereof.

The pulling rate of jig from the temperature-sensitive material for elongating the temperature-sensitive material into thread forms varies depending on the choice of temperature-sensitive material, shape and size of acicular projections formed, and the like. The rate is normally about 1 mm/minute to about 100 mm/minute, with the lower limit being preferably about 2 mm/minute or more, more preferably about 5 mm/minute or more, and particularly preferably about 10 mm/minute or more, and the upper limit being preferably about 50 mm/minute or less, more preferably about 20 mm/minute or less. In a particularly preferable aspect, the rate is about 2 to about 20 mm/minute or about 10 mm/minute to about 50 mm/minute.

As the jig and the temperature-sensitive material continue to be pulled apart at a constant rate, the temperature-sensitive material in a viscoplastic state elongated into thread forms is cut to form an acicular projection. By elongating the temperature-sensitive material in a viscoplastic state into thread forms of predetermined length, then discontinuing the pulling of the jig from the temperature-sensitive material or decelerating the pulling, the temperature-sensitive material elongated into thread forms is smoothly cut, thus ensuring more accurate formation of chipping-free acicular projections at the tips thereof, and with less deformation of the tips. In this operation, the rate of pulling the jig apart from the temperature-sensitive material is preferably in the range from 0 mm/minute to half the pulling rate of the jig from the temperature-sensitive material during elongation of the temperature-sensitive material into thread forms. When the rate is too fast beyond this range, an effect to promote the cutting of the temperature-sensitive material elongated into thread forms is unlikely to be afforded. When pulling of the jig from the temperature-sensitive material is stopped, the material is cut spontaneously because of the surface tension of the temperature-sensitive material and the like, and settles as an acicular projection, though the repose time varies depending on the kind of the temperature-sensitive material. For example, when the temperature-sensitive material is an aliphatic polyester such as polylactic acid, and when the material is kept still for about 1 to 10 seconds, the temperature-sensitive material in thread forms is cut, and settles as an acicular projection.

The processes described above enable production of microneedles of high specification reliability wherein the individual acicular projections have chipping-free uniform tips.

After formation of the acicular projections, the temperature-sensitive material may be cooled as required.

In the present invention, for correcting any irregularity of the tips of the acicular projections, adjusting their length, and other purposes, it is possible to immerse the tips of the acicular projections in a solvent that dissolves the temperature-sensitive material, to uniformly cut the tips of the acicular projections, or to increase the jig temperature to melt and cut the temperature-sensitive material, while the acicular projections formed upon pulling of the jig from the temperature-sensitive material are in contact with the jig (i.e., in a state wherein the temperature-sensitive material is elongated into thread forms), and the like.

Any solvent can be used, as far as it is capable of dissolving the temperature-sensitive material during dipping of the tips of the acicular projections therein. When the temperature-sensitive material is a resin, examples of useful solvents include halogenated hydrocarbons such as chloroform and methylene chloride, organic solvents such as ethyl acetate, acetone, tetrahydrofuran, hexane, and toluene, and water. The temperature for dissolution may be at any level below the boiling point of the solvent used, with preference given to room temperature.

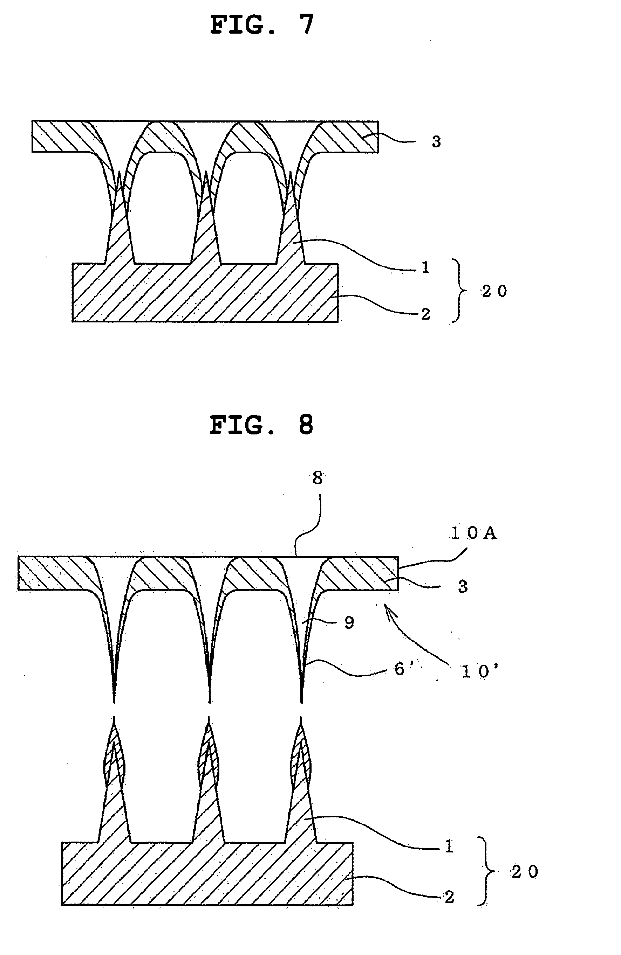

FIGS. 6 to 8 show aspects wherein a microneedle having hollow acicular projections is produced. FIG. 6 shows a second process, wherein the tips of the heated jig (tips of acicular projections 1 of microarray 20) penetrate the layer of the temperature-sensitive material 3 and stop outside thereof, the apexes of the tips being exposed to the outside atmosphere.
For this reason, the procedure of a third process, as shown in FIG. 7, suppresses the adhesion of the temperature-sensitive material in a viscoplastic state to the apexes of the acicular projections 1 of the microarray 20 during pulling of the jig from the temperature-sensitive material. As a result, as shown in FIG. 8, a microneedle 10' having hollow acicular projections 6' can be obtained. Hence, there are openings 8 opposite to the face of the substrate 10A on which the acicular projections 6' are present, with a hollow portion 9 entering from each opening into each acicular projection 6' formed.

In the production method of microneedle of the present invention, the length and thickness of the acicular projections can be adjusted optionally by adjusting the duration of contact (heating time) of the heated jig and the temperature-sensitive material, jig size (area in contact with temperature-sensitive material), the pulling rate from temperature-sensitive material and the like. Generally, it is preferable to set the acicular projection length (longitudinal length) between 100 and 500 µm. Although the thickness (outer diameter) of the root of each acicular projection is not subject to limitations, it is significantly influenced by jig size. Therefore, it is desirable to adjust the jig size as appropriate.

With respect to the acicular projections formed, at least the root of each thereof has a cross section corresponding to that of the acicular projections of the jig, and the tip has a pointed shape, with an arc extending therefrom to the root (see FIG. 14(b) and the like). As such, the shape of the projection ensures easy insertion thereof into the skin, enabling localized destruction of the stratum corneum as desired.

Although at least one acicular projection is sufficient, multiple projections may be formed as required.

As shown in FIGS. 5 and 8, the method of the present invention makes it possible to easily produce microneedles 10, 10' of high specification reliability wherein a plurality of acicular projections are arranged on a tabular substrate (in particular, a microneedle wherein a plurality of acicular projections are arranged in an array), and wherein the individual acicular projections have chipping-free uniform tips.

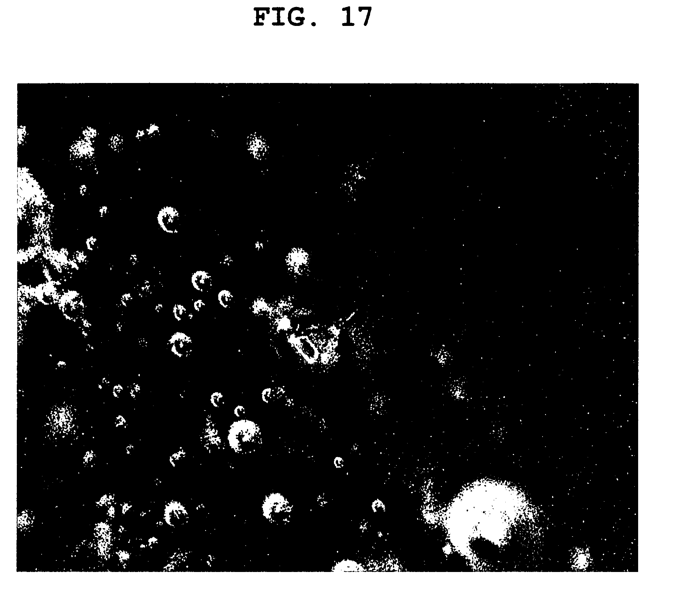

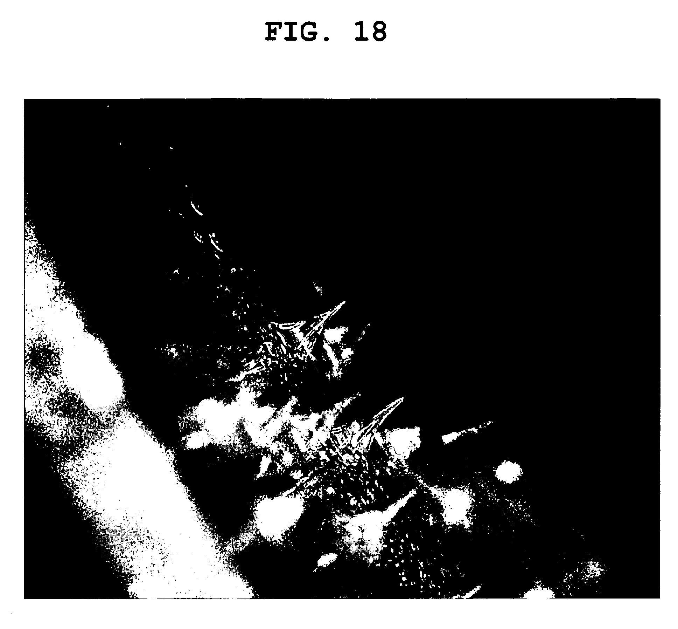

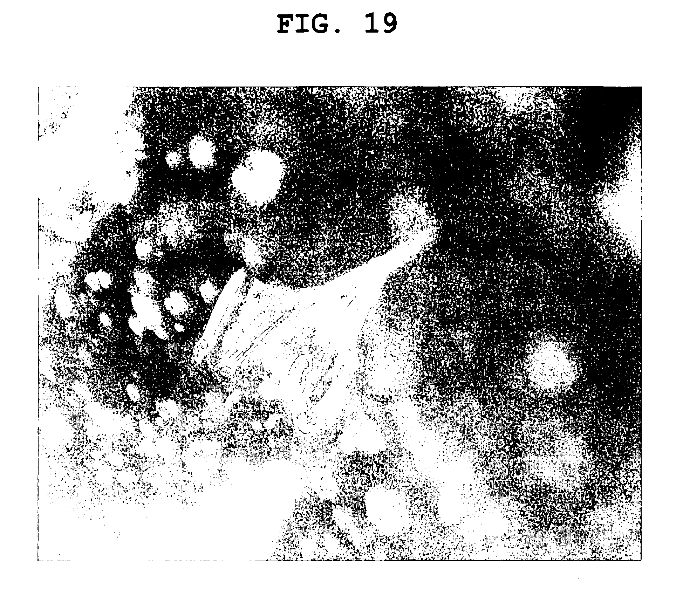

In the method of production of the present invention, the external shape of the jig is generally a column, prism, cone, pyramid, truncated cone, truncated pyramid and the like, but the jig may have a U-shaped or S-shaped cross-section. Using such a jig in the method of production of the present invention, a microneedle having an acicular projection at least whose root has a circular, polygonal (e.g., triangular, square (FIG. 17) and the like), U-shaped (FIGS. 18, 19), S-shaped or other cross section can be produced. FIG. 17 is a light photomicrograph of an acicular projection whose root has a square cross section, formed from a biodegradable resin.
FIGS. 18 and 19 are light photomicrographs of an acicular projection whose root has a U-shaped cross section, formed from a biodegradable resin.

As used herein, the term "the cross-sectional shape of an acicular projection of a microneedle or jig" refers to the shape of a cross-section perpendicular to the axial line of the projection.

The microneedle obtained by the method of the present invention is a member for use as an auxiliary in transdermal administration of drugs. The choice of drug is not limited to therapeutic drugs for diseases, but generally encompasses substances that exhibit bioactivity in living organisms. Examples include, but are not limited to, antibiotics, antiviral agents, analgesics, anesthetics, anorectics, antiarthritic agents, antidepressants, antipsychotics, antihistamine agents, anti-inflammatory agents, antitumor agents, vaccines, including DNA vaccines, adjuvants, allergic antigens, biological products, proteins, peptides and fragments thereof, and the like. When a drug is administered in the form or a liquid prepared by dissolving or dispersing the drug in a solvent, the solvent can be chosen optionally from among biologically compatible solvents. Examples include, but are not limited to, distilled water, physiological saline, glucose aqueous solutions, alcohols, propylene glycol, vegetable oils and the like, and combinations thereof. Furthermore, additives such as solubilizers (e.g., polyethylene glycol, propylene glycol, D-mannitol, ethanol and the like), surfactant (e.g., sodium lauryl sulfate and the like), isotonizing agents (e.g., sodium chloride and the like), buffering agents (e.g., phosphoric acid, acetic acid and the like), and preservatives (e.g., p-hydroxybenzoic acid ester and the like) can also be added.

When a microneedle obtained by the method of the present invention has an acicular projection wherein the cross-sectional shape of at least the root thereof is U-shaped or S-shaped, a fine pit (groove) is formed in a side of the root (see FIGS. 18 and 19), so a drug reservoir is formed in the pit (groove) during administration of the drug, enabling more efficient administration of the drug.

When a microneedle obtained by the method of production of the present invention has a hollow acicular projection, more efficient administration of a drug is possible because the hollow portion can retain the drug.

A drug-containing microneedle can be prepared by previously mixing the drug in a temperature-sensitive material, and preparing the microneedle as specified above. Such a drug-containing microneedle enables the release of the drug from the microneedle by penetration into the body (skin). In particular, with the use of a biodegradable resin (polylactic acid, polyglycolic acid, lactic acid-glycolic acid copolymer and the like) or a polysaccharide as the temperature-sensitive material, the microneedle is decomposed upon penetration into the body (skin), whereby the drug is smoothly released into the body.

The present invention is hereinafter described in further detail by means of the following examples. The present invention is not limited by the examples, but can be embodied with modifications that fall within the scope of the invention described herein, all of which are encompassed in the technical scope of the present invention.

### Examples

### Example 1 (Production of polylactic acid resin microneedle)

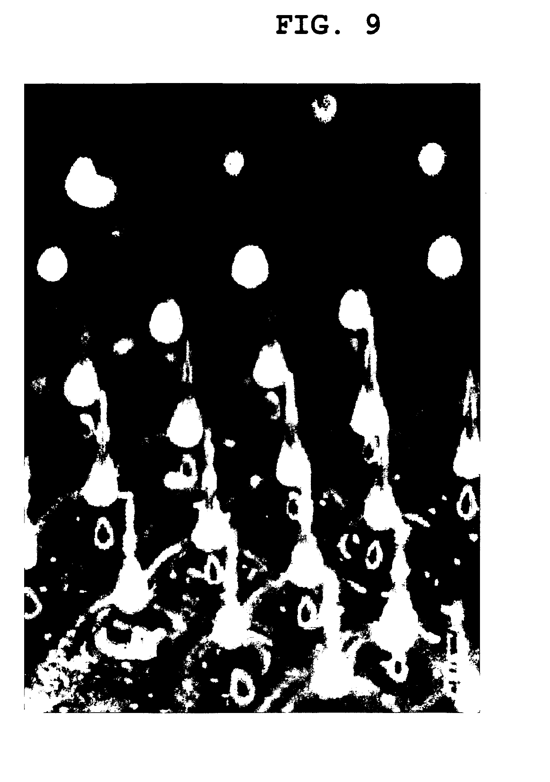

A silicon microneedle (a jig wherein 12 × 12 prismatic microneedles with 100 µm sides, 300 µm in length, were placed in array on 7 mm × 7 mm square substrate) was placed on a hot plate and heated at 80°C.
A polylactic acid (Mw: 10,000) was thermally molten on an aluminum substrate and cooled to yield a tabular sheet.
The polylactic acid sheet was placed on the support pillar of a Shimadzu rheometer (EZ-TEST) with its top down. The support pillar was pulled down to bring the jig into contact with the sheet to make the temperature-sensitive material at the contact portion viscoplastic. The support pillar was kept at that position for 5 seconds, and then pulled up at a rate of 5 mm/minute.
When the resin sheet was pulled up, a resin microarray (microneedle) having acicular projections with nearly uniform length of 300 µm was obtained (FIG. 9).

### Example 2 (Production of maltose microneedle)

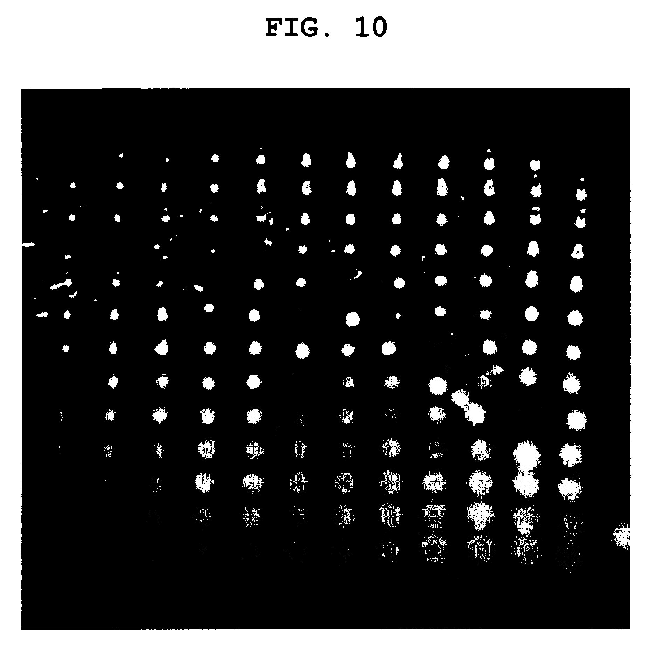

Maltose was thermally molten on an aluminum substrate and cooled to yield a tabular sheet. Using this sheet, a microarray (microneedle) was prepared by the same method as Example 1. The hot plate heating temperature was 150°C, the duration of contact of the jig with the sheet was 5 seconds, and the support pillar raising rate was about 50 mm/minute.
When the resin sheet was pulled up, a microarray of maltose having microneedles of uniform length was obtained (FIG. 10).

### Example 3 (Production of polylactic acid resin microneedle)

A silicon microneedle (a jig wherein 12 × 12 quadrangular prismatic microneedles with 100 µm sides, 300 µm in length, were placed in array on 7 mm × 7 mm square substrate) was placed on a hot plate and heated at 80°C.
A polylactic acid (Mw: 10,000) was thermally molten on an aluminum substrate and cooled to yield a tabular sheet.
The polylactic acid sheet was placed on the support pillar of a Shimadzu rheometer (EZ-TEST) with the top on. The support pillar was pulled down to bring the jig into contact with the sheet, and the support pillar was kept at that position for 7 seconds. Subsequently, the support pillar was pulled up by about 2 mm at a rate of 10 mm/minute to form a thread-like polylactic acid resin between the pillar and the jig. Subsequently, the support pillar was kept stationary for about 3 seconds; the thread-like polylactic acid resin became thinner and cut, and settled as acicular projections. As a result, a resin microarray (microneedle) having acicular projections with a uniform length of about 150 µm was obtained.

### INDUSTRIAL APPLICABILITY

The method of microarray or microneedle production of the present invention is of high practical value because of its simplicity that enables the preparation of desired microneedles using various biocompatible resins. Furthermore, the method enables easy production of preparations of high transdermal absorbability by adding a drug into a biocompatible resin (in particular, biodegradable resin).
This application is based on a patent application No. 2007-018006 filed in Japan, the contents of which are incorporated in full herein by this reference.

## Claims

1. A method of producing a microneedle from a temperature-sensitive material that exhibits thermoplastic deformation, comprising:
heating a jig to a temperature above the level for viscoplastic transformation of the temperature-sensitive material, bringing the jig into contact with the temperature-sensitive material, thereafter pull the jig apart from the temperature-sensitive material, to elongate the temperature-sensitive material bonded to the jig, and to form acicular projections.

2. A method of producing a microneedle, comprising the steps (i) to (iv) below:
(i) the step of setting a jig at a temperature above the level for a viscoplastic state of a temperature-sensitive material that exhibits thermoplastic deformation,
(ii) the step of bringing the jig into contact with the temperature-sensitive material to make the contact portion of the temperature-sensitive material viscoplastic,
(iii) the step of pulling the jig from the temperature-sensitive material to elongate the temperature-sensitive material in the viscoplastic state in the form of threads, and
(iv) the step for discontinuing the pulling or decelerating the pulling the jig from the temperature-sensitive material to cut the threads of the temperature-sensitive material and form acicular projections.

3. The method of claim 1 or 2, wherein either one of the temperature-sensitive material and the jig is placed on an upper portion of a straight line perpendicular to the horizontal plane, and the other on a lower portion, to allow the formation of acicular projections in the gravitational direction.

4. The method of claim 1 or 2, wherein the temperature-sensitive material and the jig are arranged side by side on a straight line parallel to the horizontal plane, to allow the formation of acicular projections in the horizontal direction.

5. The method of any one of claims 1 to 4, wherein the jig is a microneedle of silicon or metal.

6. The method described in any one of claims 1 to 5, wherein the jig temperature is not lower than the glass transition point or melting point of the temperature-sensitive material.

7. The method of any one of claims 1 to 6, wherein heating of the temperature-sensitive material by the jig lasts for 1 second to 1 minute.

8. The method of any one of claims 1 to 7, wherein the pulling rate of the jig from the temperature-sensitive material for elongating the temperature-sensitive material into threads is 1 mm/minute to 100 mm/minute.

9. The method of any one of claims 1 to 7, wherein the pulling rate of the jig from the temperature-sensitive material for elongating the temperature-sensitive material into threads is 2 mm/minute to 20 mm/minute.

10. The method of any one of claims 2 to 9, wherein the pulling rate of the jig from the temperature-sensitive material for cutting the threads of the temperature-sensitive material is in the range from 0 mm/minute to half the pulling rate of the jig from the temperature-sensitive material for elongating the temperature-sensitive material into thread forms.

11. The method of any one of claims 2 to 9, wherein the pulling of the jig from the temperature-sensitive material is discontinued to cut the threads of the temperature-sensitive material.

12. The method of any one of claims 1 to 11, wherein the temperature-sensitive material is a biodegradable resin or a polysaccharide.

13. The method of claim 12, wherein the biodegradable resin is a polyfatty acid ester.

14. The method of claim 13, wherein the polyfatty acid ester is polylactic acid, polyglycolic acid, or a lactic acid-glycolic acid copolymer.

15. The method of claim 12, wherein the polysaccharide is maltose.

16. The method of any one of claims 1 to 15, wherein the temperature-sensitive material has been shaped in a tabular form in advance.

17. The method of any one of claims 1 to 16, further comprising immersing and dissolving the tips of the acicular projections in a solvent that dissolves the acicular projections.

18. A microneedle of temperature-sensitive material obtained by the method of claim 1, each having an acicular projection whose root has a cross section corresponding to the cross section of the jig, whose tip has a pointed shape, and which has an arc extending from the tip to the root.

19. The microneedle of claim 18, wherein the cross section of the root of the acicular projection is circular, polygonal, U-shaped or S-shaped.

20. The microneedle of claim 18 or 19, wherein the temperature-sensitive material is a biodegradable resin or a polysaccharide.

21. The microneedle of claim 20, wherein the biodegradable resin is a polyfatty acid ester.

22. The microneedle of claim 21, wherein the polyfatty acid ester is polylactic acid, polyglycolic acid, or a lactic acid-glycolic acid copolymer.

23. The microneedle of claim 20, wherein the polysaccharide is maltose.

24. The microneedle of any one of claims 18 to 23, wherein acicular projections are provided on a tabular substrate.

25. The microneedle of claim 24, having a plurality of acicular projections arranged in an array.

26. The microneedle of any one of claims 18 to 25, wherein the acicular projections are hollow acicular projections.
